# EUROPEAN PATENT APPLICATION

(11) **EP 4 112 736 A1**
(43) Date of publication of application: **04.01.2023**
(21) Application number: 21275092.1
(22) Date of filing: 02.07.2021
(51) Int. Cl.: C12Q 1/60, G01N 33/52, G01N 33/92

(54) **DEVICE FOR TESTING BLOOD PLASMA**

(71) Applicant: Vital Signs Solutions Limited, Cambridge Cambridgeshire CB4 0FW (GB)
(72) Inventor: Prameen, Kalikavunkal, Cambridge, CB4 0FW (GB); Ameya, Vaidya, Cambridge, CB4 0FW (GB)
(74) Representative: Williams Powell

(57) **Abstract**

A single device for testing each of total cholesterol, HDL, and triglyceride concentrations of a whole blood sample. The device includes an inlet (10) for blood plasma and a separation element (200) in fluid communication with the inlet (10), the separation element (200) (200) including a plurality of channels (210, 220, 230), each channel allowing capillary flow of blood plasma from the inlet (10) to a respective testing region (1, 2, 3).

## Description

### Field of invention

The present invention relates to a device for testing blood plasma, and in particular to a device for testing blood plasma for cholesterol.

### Background of the invention

Cardiovascular diseases are a major cause of death worldwide. Total cholesterol (TC), triglycerides (TGL), and high density cholesterol (HDL) (sometimes referred to as "good cholesterol") are important biomarkers to ascertain the risk of suffering from cardiovascular conditions in the future. Testing regimes for the amounts of these different types of cholesterol - and the ratio between them - are commonly used to predict a patient's future health profile.

Common cholesterol test devices measure one of the three kinds of cholesterol from a single drop of blood (10µl). Some test kits integrate all the tests into one and produce the individual results from a few drops of blood (50µl). These tests kits require additional expensive equipment for the measurement and analysis.

The present application seeks to provide an improved cholesterol testing device.

### Summary of the invention

In accordance with a first aspect of the present invention, there is provided a device for testing blood plasma for cholesterol, including
an inlet for blood plasma,
a separation element in fluid communication with the inlet, wherein the separation element is formed of a material which allows capillary flow of blood plasma,
wherein the separation element includes a first channel for capillary flow of blood plasma from the inlet to a first test region, a second channel for capillary flow of blood plasma from the inlet to a second test region, and a third channel for capillary flow of blood plasma from the inlet to a third test region,
wherein the third channel includes a reactant for reacting with low density lipoprotein (LDL) cholesterol, thereby preventing it from reaching the third test region,
and wherein the first test region includes a test for identifying total cholesterol, the second test region includes a test for identifying triglycerides and the third test region includes a test for identifying high density lipoprotein (HDL) cholesterol.

The technical advantage of the present invention is the provision of a single device which is able to determine the concentration of Total cholesterol, HDL and Triglyceride concentrations from a single prick of whole blood (about 15µL) without the use of any anticoagulants.

The device is engineered to extract plasma from whole blood using a 3 layered lateral flow assay model.

Preferably, the separation element is formed from a cellulose membrane. Preferably this is substantially planar.

The separation element (which is preferably formed from a nitrocellulose membrane) may have hydrophobic and hydrophilic regions which are configured so as to define said channels and test regions, with the hydrophilic regions forming said channels and test regions.

The hydrophobic regions may be formed by including a wax on the separation element. Other methods of forming the hydrophobic regions include spray painting hydrophobic molecules (such as silanes) onto the separation element or printing melted paraffin wax using a custom-built printer.

In a preferred embodiment, the device additionally includes a filter for separating blood plasma from whole blood, the filter being located on the opposite side of the inlet to said channels, the filter including at least two filtration layers. The first filtration layer may be a polymer mesh (preferably a nylon mesh) and the second layer may be a glass fibre mesh (preferably an LF1 membrane). In a particularly preferred embodiment, the filter has a tapered part in communication with said inlet.

The individual pores in the nylon mesh acts as a capillary matrix to transfer the fresh whole blood onto a tapering LF1 membrane below it. The LF1 membrane separates the whole blood into plasma and blood cells, and the tapering end conveys the plasma separated onto the nitrocellulose membrane. The tapering section allows the plasma to accumulate and flush into the nitrocellulose membrane below it.

In accordance with a second aspect of the invention, there is provided a device for testing blood plasma for cholesterol, including
an inlet for blood plasma,
a flow element in fluid communication with the inlet, wherein the flow element comprises one or more channels and is formed of a material which allows capillary flow of blood plasma,
a testing site for testing the blood plasma in fluid communication with the flow element, and
a filter for separating blood plasma from whole blood, the filter being located on the opposite side of the inlet to said channels, the filter including at least two filtration layers,
wherein the filter has a tapered part in communication with said inlet.

Preferably the filtration layers are as defined in the first aspect of the invention.

### Specific description

A number of preferred embodiments of the invention will now be described, with reference to and as illustrated in the accompanying drawings, in which:
Figure 1 is an exploded view of an embodiment of the device;
Figure 2 is a plan view of an embodiment of a separation element (200) suitable for use with the device of Figure 1.

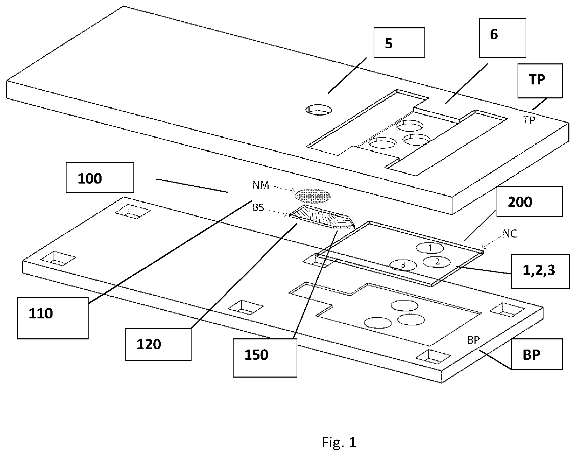

The device as shown in Figure 1 has a top piece (TP) and a bottom piece (BP), each of which is substantially planar. The top and bottom pieces (TP, BP) are configured to interlock with one another such that a unitary body or housing is formed when they are locked together.

Sandwiched between the top and bottom pieces (TP, BP) are a filter (100) and a separation element (200), which are received within a recess of the bottom piece (BP). Disposed in the top piece (TP) is a blood inlet port (5), through which a user can deposit a sample of whole blood (i.e. red blood cells, white blood cells, platelets, and blood plasma) into the device for assay. Once deposited into the inlet port (5), blood plasma from the sample travels through the filter (100) and the separation element (200) by capillary action to reach each of three test regions (1, 2, 3). The first test region (1) includes a test for identifying total cholesterol, the second test region (2) includes a test for identifying triglycerides, and the third test region (3) includes a test for identifying high density lipoprotein (HDL) cholesterol. The top piece (TP) also includes a plurality of additional ports (6) which are sized and positioned to give access to each of the three test regions (1, 2, 3).

The filter (100) is disposed between, and in fluid communication with each of, the blood inlet port (5) and the separation element (200). The function of the filter (100) is to separate the blood plasma from a volume of whole blood deposited into the blood inlet port (5).

The filter (100) comprises first and second filtration layers (110, 120). The first filtration layer (110) is located adjacent the blood inlet port (5) and is formed of a polymer mesh, such as a nylon mesh. Pores in the polymer mesh of the first filtration layer (110) act as a capillary, enabling whole blood to flush evenly across the filter (100). The second filtration layer (120) is located beneath the first layer (110) and is formed of a glass fibre mesh, such as an LF1 membrane. The second filtration layer (120) separates blood plasma from the whole blood laterally across the glass fibre mesh. An LF1 membrane, in particular, enables a high retention of blood cells compared with other fibre meshes.

The second filtration layer (120) has a first broad end and a second end which narrows to form a tapered part (150). The tapered part (150) is in communication with an inlet (10) at the separation element (200). The broad end of the second filtration layer (120) is shaped and sized to allow all the blood from the first filtration layer (110) to be transferred to the second filtration layer (120). The tapered part (150) at the second end is shaped to constrict the blood cells in the glass fibre mesh and concentrate blood plasma at the tip of the taper, where it is then transferred onto the separation element (200) via the inlet (10).

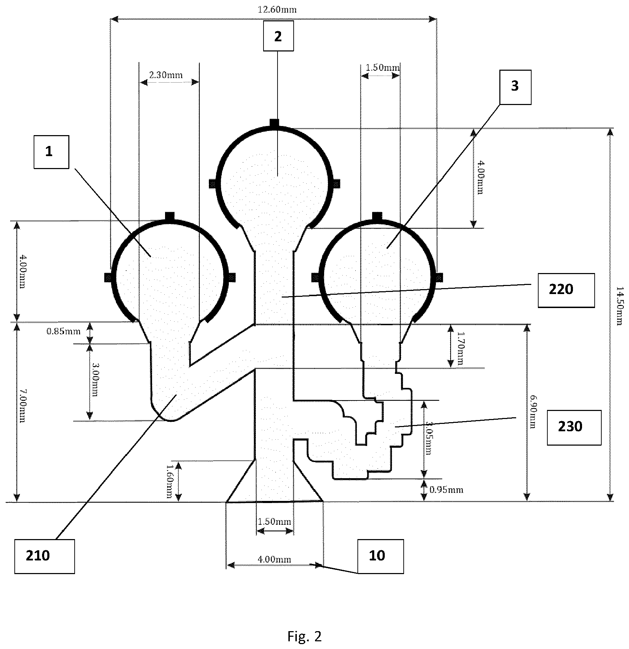

Referring now to Figure 2, an embodiment of the separation element (200) will be described. The separation element (200) is substantially planar and formed of a material which allows capillary flow of blood plasma, such as a nitrocellulose membrane.

As described above, an inlet (10) enables a flow of blood plasma into the separation element (200) from the filter (100). The separation element (200) (200) comprises first, second, and third elongate channels (210, 220, 230) configured to effect capillary flow of blood plasma from the inlet (10) to each of the first, second, and third test regions (1, 2, 3). The separation element (200) has hydrophobic and hydrophilic regions which define the channels and test regions (1, 2, 3), with the hydrophilic regions forming said channels and test regions (1, 2, 3). The hydrophobic regions are formed by the addition of a hydrophobic material, such as a wax.

Each of the channels (210, 220, 230) has a different degree of tortuousness, thereby differently effecting the flow of blood plasma therethrough. The second channel (220) is substantially straight. The first channel (210) includes a corner. The third channel (230) is more tortuous than each of the first and second channels (210, 220), comprising a plurality of corners which slow down capillary flow of the blood plasma to the third test region (3).

The third channel (230) is also longer than each of the first and second channels (210, 220) in order to increase the time taken for blood plasma to reach the third test region (3). Figure 2 illustrates the dimensions of a typical embodiment of the separation element (200). For the embodiment illustrated in Figure 2, a volume of 3.5µL of blood plasma is required to wick through the entire hydrophilic region of the separation element (200).

The third channel (230) further includes a reactant for reacting with low density lipoprotein (LDL) cholesterol (for example dextran sulphate, phosphotungistic acid, magnesium sulphate, magnesium chloride, or a combination thereof), thereby preventing it from reaching the third test region (3).

The device is advantageously physico-chemical and requires no electronics to generate a readable output, nor does it generate any energy of note itself. It utilises the ability of liquid (blood or plasma) to move across porous material by capillary forces.

The operation of the device according to the embodiments described above is set out sequentially below:
1. 15µL of whole blood (from a fingerprick) is transferred with a calibrated micropipette from the finger of a human onto the blood inlet port (5).
2. The deposited whole blood is quickly spread over a circular area (Ø 6mm) of the first filtration layer (110), which comprises a high-porosity nylon mesh membrane, and descends by gravity and capillary forces onto the second filtration layer (120), which comprises an LF1 glass fibre membrane. Red blood cells, white blood cells, and platelets are then separated from the whole blood as the blood starts moving laterally across the second filtration layer (120).
3. The tip of the tapered part (150) of the second filtration layer (120) is in communication with the separation element (200), which comprises a nitrocellulose membrane, via inlet (10). Blood plasma (i.e. whole blood depleted of blood cells and platelets), continues flowing on the separation element (200) by capillary forces on the nitrocellulose membrane and the flow is split into three channels (210, 220, 230).
4. Plasma flowing through the first channel (210) reaches the first test region (1) within 2-3 minutes, and all cholesterol and cholesterol esters molecules present in the plasma react with reagents infused at the test area to produce a distinct purple colour, the intensity of which is proportional to the concentration of triglycerides in the plasma sample.
5. Plasma flowing through the second channel (220) reaches the second test region (2) within 2-3 minutes, and triglycerides present in the plasma sample react with reagents infused at the test area to produce a distinct purple colour, the intensity of which is proportional to the concentration of total cholesterol in the plasma sample.
6. The third channel (230) is infused with a reactant that reacts with low-density lipoprotein (LDL) cholesterol, thereby preventing it from reaching the third test region (3). Therefore, only high-density lipoproteins (HDL) continue flowing, enabling the detection of the HDL portion of the blood sample at the third test region (3).
7. Plasma flowing through the third channel (230) reaches the third test region (3), usually within 3-4 minutes, and only HDL present in the plasma sample reacts with the reagents infused at the test area to produce a distinct purple colour, the intensity of which is proportional to the concentration of HDL in the plasma sample.
8. After 6 minutes has elapsed from the deposition of the blood sample - being sufficient time for the optimum purple colour to have developed in each of the test regions (1, 2, 3) - a colourimetric analysis can be conducted.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. A device for testing blood plasma for cholesterol, including
an inlet (10) for blood plasma,
a separation element (200) in fluid communication with the inlet (10), wherein the separation element (200) is formed of a material which allows capillary flow of blood plasma,
wherein the separation element (200) includes a first channel (210) for capillary flow of blood plasma from the inlet (10) to a first test region (1), a second channel (220) for capillary flow of blood plasma from the inlet (10) to a second test region (2), and a third channel (230) for capillary flow of blood plasma from the inlet (10) to a third test region (3),
wherein the third channel (230) includes a reactant for reacting with low density lipoprotein (LDL) cholesterol, thereby preventing it from reaching the third test region (3),
and wherein the first test region (1) includes a test for identifying total cholesterol, the second test region (2) includes a test for identifying triglycerides and the third test region (3) includes a test for identifying high density lipoprotein (HDL) cholesterol.

2. A device as claimed in claim 1, wherein the separation element (200) is formed from a cellulose membrane.

3. A device as claimed in claim 1 or 2, wherein the separation element (200) is substantially planar.

4. A device as claimed in any preceding claim, wherein the separation element (200) has hydrophobic and hydrophilic regions which are configured so as to define said channels and test regions (1, 2, 3), with the hydrophilic regions forming said channels and test regions (1, 2, 3).

5. A device as claimed in claim 4, including a wax on the separation element (200) which forms the hydrophobic regions.

6. A device as claimed in any preceding claim, wherein the reactant for the third channel (230) includes dextran sulphate, phosphotungistic acid, magnesium sulphate, magnesium chloride, or a combination thereof.

7. A device as claimed in any preceding claim, wherein the third channel (230) is longer than the first channel (210) and longer than the second channel (220) in order to increase the time taken for blood plasma to reach the third test region (3).

8. A device as claimed in any preceding claim, wherein the third channel (230) includes a plurality of corners along its path, whereby the corners slow down capillary flow of the plasma to the third test region (3).

9. A device as claimed in claim 8 wherein the third channel (230) includes from 8 to 20 corners.

10. A device as claimed in any preceding claim, additionally including a filter (100) for separating blood plasma from whole blood, the filter (100) being located on the opposite side of the inlet (10) to said channels, the filter (100) including at least two filtration layers.

11. A device as claimed in claim 10, wherein the first filtration layer is a polymer mesh.

12. A device as claimed in claim 10 or 11, wherein the second layer (120) is a glass fibre mesh.

13. A device as claimed in any of claims 10 to 12 wherein the filter (100) has a tapered part (150) in communication with said inlet (10).

14. A device as claimed in any of claims 10 to 13, which device is housed in a housing having 4 ports, one port to give access to the filter (100) and three ports to give access to the three test regions (1, 2, 3).

15. A device for testing blood plasma for cholesterol, including
an inlet (10) for blood plasma,
a flow element in fluid communication with the inlet (10), wherein the flow element comprises one or more channels and is formed of a material which allows capillary flow of blood plasma,
a testing site for testing the blood plasma in fluid communication with the flow element, and
a filter (100) for separating blood plasma from whole blood, the filter (100) being located on the opposite side of the inlet (10) to said channels, the filter (100) including at least two filtration layers,
wherein the filter (100) has a tapered part (150) in communication with said inlet (10).

16. A device as claimed in claim 15, wherein the first filtration layer is a polymer mesh.

17. A device as claimed in claim 15 or 16, wherein the second layer (120) is a glass fibre mesh.
